# EUROPEAN PATENT SPECIFICATION

(11) **EP 3 177 150 B1**
(45) Date of publication and mention of the grant of the patent: **23.12.2020**
(21) Application number: 15750345.9
(22) Date of filing: 06.08.2015
(51) Int. Cl.: A23C 9/20, A61K 31/525, A61K 31/7004, A61K 35/744, A61K 35/745, A61K 35/747, A61K 31/592, A61K 31/593, A61K 33/30, A61P 3/10

(54) **A COMBINATION OF VITAMIN D AND ZINC AND ITS USE**
KOMBINATION AUS VITAMIN D UND ZINK UND DEREN VERWENDUNG
COMBINAISON DE VITAMINE D ET DE ZINC ET SON UTILISATION

(30) Priority: 08.08.2014 EP 14180396; 08.08.2014 EP 14180399; 08.08.2014 EP 14180401; 08.08.2014 EP 14180403; 22.05.2015 EP 15168958; 05.06.2015 EP 15170881
(43) Date of publication of application: 14.06.2017
(73) Proprietor: Société des Produits Nestlé S.A., 1800 Vevey (CH)
(72) Inventor: SILVA ZOLEZZI, Irma, CH-1084 Carrouge (CH); MINEHIRA CASTELLI, Kaori, CH-1804 Corsier-sur-Vevey (CH); SAKAMOTO, Kei, CH-1009 Pully (CH)
(74) Representative: Gagliardi, Tatiana
(86) International application number: PCT/EP2015/068183
(87) International publication number: WO 2016/020485

(56) References cited:
- CN-A- 104 509 849
- US-A1- 2002 155 163
- US-A1- 2006 088 574
- US-A1- 2010 178 281
- US-A1- 2012 107 291
- US-A1- 2013 273 015
- EWELINA ROGOZINSKA ET AL: "Nutritional Manipulation for the Primary Prevention of Gestational Diabetes Mellitus: A Meta-Analysis of Randomised Studies", PLOS ONE, vol. 10, no. 2, 26 February 2015 (2015-02-26), page e0115526, XP055211940, DOI: 10.1371/journal.pone.0115526
- RAAKEL LUOTO ET AL: "Impact of maternal probiotic-supplemented dietary counselling on pregnancy outcome and prenatal and postnatal growth: a double-blind, placebo-controlled study", BRITISH JOURNAL OF NUTRITION, vol. 103, no. 12, 4 February 2010 (2010-02-04), pages 1792-1799, XP055030014, ISSN: 0007-1145, DOI: 10.1017/S0007114509993898

## Description

### Field of the invention

The invention relates to a combination of vitamin D and zinc for use in improving insulin sensitivity and/or treating or preventing an impaired glucose tolerance and/or type II diabetes and/or gestational diabetes mellitus and/or preventing a condition associated with any of the forgoing in a subject.

### Background

Glucose is one of a key nutrient which gives vital energy to living cells in our organs. The glucose can be derived from food and also de novo synthesized by the liver and kidney during a fasting condition. During digestion carbohydrates from glucidic foods are transformed into glucose which is passed through the intestinal wall into the bloodstream. This influx of glucose into the bloodstream results in an increase in the blood glucose level.

The level of blood glucose is tightly regulated by a hormone, insulin. As the blood glucose level increases insulin is secreted by the pancreas. Insulin is the hormone responsible for bringing glucose to insulin-sensitive tissues such as liver, muscle and adipose tissues, thus lowering the blood glucose level in the blood However, some subjects do not respond adequately to insulin. These subjects have an insulin resistance or a low insulin sensitivity. This may be because insulin receptors in the insulin-sensitive tissues do not respond adequately to insulin. In response to an inadequate response to insulin the pancreas may secrete more insulin to compensate. These subjects maybe considered as having an impaired glucose tolerance (hereinafter IGT). Eventually, the pancreas may fail to keep up with the body's increased need for insulin, leading to type II diabetes. According to the World Health Organization, at least 171 million people worldwide suffered from type II diabetes. Its incidence is increasing rapidly, and it is estimated that by 2030, this number will almost double. An impaired glucose tolerance (also referred to as prediabetes) is even more common, and and more than one out of three adults have been estimated "prediabetic" or as having an IGT in the United States.

Insulin resistance (a low insulin sensitivity) occurs in pregnant subjects. This is due to hormonal changes that help to ensure the transfer of nutrients from the pregnant subject to the fetus. As described above, in response to insulin resistance the pancreas may secrete more insulin, and as described above, eventually the pancreas may fail to keep up with the body's increased need for insulin. Any degree of glucose intolerance with onset or first recognition during pregnancy is referred to as Gestational Diabetes Mellitus (hereinafter GDM). GDM affects up to 15% of pregnant women worldwide, and in India alone an estimated 4 million women have GDM.

An impaired glucose tolerance and/or type II diabetes are associated with an increased risk of a variety of diseases for example Cardio Vascular Disease (hereinafter CVD). CVD accounts for approximately 20% of all annual worldwide deaths, and remains one of the leading causes of death in both the developed and developing world. GDM is a pregnancy disorder that can increase the risk of a number of maternal-fetal conditions.

Dietary and lifestyle changes, including healthier dietary habits and increased exercise, can be very efficient in improving an IGT and in preventing type II diabetes, however, patient compliance can be problematic. Drugs such as biguanides and thiazolidinediones are available for improving insulin sensitivity or type II diabetes. However, many of these have unwanted side effects and cannot be used in pregnancy. Moreover, there is no practice of giving such drugs to prevent IGT.

Document CN 104509849 A describes a nutrient for control weight gaining during gestation period of pregnant and lying-in women is characterized by being composed of following nutritional components: fat, glucose, DHA, proteins, inorganic salts (including iron, magnesium, calcium, zinc, iodine, phosphorus, selenium and manganese), vitamin (including vitamin A, vitamin D, vitamin B1, vitamin B2, nicotinic acid, folic acid, and vitamin C).

Document US2010/178281 describes the use of probiotic bacteria in the manufacture of a composition for administration to a woman in at least the third trimester of pregnancy for prevention of gestational diabetes, normalising plasma glucose concentration and/or increasing insulin sensitivity.

Document Raakel Luoto et al. British Journal of Nutrition, vol. 103, no.12, 4 February 2010, pages 1792-1799, XP055030014 describes a study to determine the safety and efficacy of perinatal probiotic supplemented dietary counselling by evaluating pregnancy outcome and fetal and infant growth during the 24months' follow up.

Document US2006/088574 A1 describes nutritional supplements preferably including slow digesting carbohydrates that render them effective in maintaining blood glucose levels at normal blood glucose levels. Such nutritional supplements can be employed with humans that are diabetic, borderline diabetic, pregnant and/or lactating, geriatric humans and humans that have or develop other glucose intolerance or cardiovascular disease.

Accordingly, there is a need to find alternative ways to improve insulin sensitivity and to treat or prevent an IGT and/or type II diabetes, and/or GDM, and/or to prevent a condition associated with any of the foregoing in a subject.

Surprisingly the inventors have found that a combination of vitamin D and zinc may be used to enhance glucose uptake in mammalian cells. Unexpectedly it was found that vitamin D and zinc, when used in combination, have a more than additive effect or synergistic effect on the enhancement of glucose uptake in mammalian cells, in particular mammalian muscle cells.

### Summary of the Invention

The invention is set out in the claims.

A combination of zinc and vitamin D, or a composition comprising vitamin D and zinc, is used to improve insulin sensitivity and/or to treat or prevent an impaired glucose tolerance and/or type II diabetes and/or GDM and/or to prevent a condition associated with any of the foregoing in a subject e.g. a mammal such as a human, cat, or dog. In the case of GDM the subject may be a pregnant mammal or the offspring of a pregnant mammal.

A low insulin sensitivity, an IGT, type II diabetes and/or GDM are associated with an increased risk of a variety of conditions. A low insulin sensitivity, an impaired glucose tolerance, and/or type II diabetes can, for example, be associated with one or more of CVD, strokes, circulatory problems that in extreme cases can lead to amputation, diabetic retinopathy, kidney failure, hearing loss, and impaired cognitive ability.

GDM can, for example, be associated with preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity, immediately after birth and later in the life of the offspring, and an increased risk for the mother of having or developing type 2 diabetes immediately after birth and later in life.

By improving insulin sensitivity and/or treating or preventing an IGT and/or type II diabetes and/or GDM, a combination of zinc and vitamin D or a composition comprising vitamin D and zinc, may also be used to treat or prevent associated conditions such as these listed herein in a subject.

A combination of zinc and vitamin D may be even more effective at improving insulin sensitivity and/or treating or preventing IGT and/or type II diabetes and/or GDM in a subject, when used in combination with myo-inositol (*cis*-1,2,3,5-*trans*-4,6-cyclohexanehexol) and/or one or more probiotic, and/or one or more of vitamin B2, B6, and B12. Accordingly, a composition comprising zinc and vitamin D may optionally also comprise myo-inositol (*cis*-1,2,3,5-*trans*-4,6-cyclohexanehexol) and/or one or more probiotic, and/or one or more of vitamin B2, B6, and B12. Said composition may be more effective at improving insulin sensitivity and/or treating or preventing IGT and/or type II diabetes and/or GDM in a subject.

A combination of zinc and vitamin D, and optionally myo-inositol and/or one or more probiotics and/or one or more of vitamin B2, B6, and B12, optionally comprised in a composition, may be administered enterally to a subject.

The combination of zinc and vitamin D, and optionally myo-inositol and/or one or more probiotics and/or one or more of vitamin B2, B6, and B12, may be administered or employed in any form suitable for ingestion by a subject. It may be comprised in a composition comprising zinc and vitamin D, and one or more other optional ingredient as laid out herein. Said composition may for example be a nutritional product, a food product, a functional food product, a healthy ageing product, a dairy product, a nutritional supplement, a pharmaceutical formulation, a beverage product, or a pet food product.

The combination of zinc and vitamin D and/or a composition comprising vitamin D and zinc may be used in the manufacture of a medicament for use to improve insulin sensitivity and/or to treat or prevent an impaired glucose tolerance and/or type II diabetes and/or GDM and/or to prevent a condition associated with any of the foregoing in a subject.

### Drawings

FIG. 1 - Chart illustrating the effect of zinc chloride, in the absence of insulin, on glucose uptake in L6 differentiated muscle cells after a 2 hour incubation.
FIG. 2 - Chart illustrating the effect of 1,25-dihydroxyvitamin D3, in the presence and absence of insulin, on glucose uptake in L6 differentiated muscle cells after a 2 hour incubation.
FIG. 3 - Chart illustrating the effect of the combination of zinc chloride and 1,25-dihydroxyvitamin D3, in the presence and absence of insulin, on glucose uptake in L6 differentiated muscle cells after a 2 hour incubation.
FIG. 4 - Chart illustrating the effect of zinc chloride, in the presence and absence of insulin, on glucose uptake in insulin resistant L6 differentiated muscle cells after a 2 hour incubation.
FIG. 5 - Chart illustrating the effect of 1,25-dihydroxyvitamin D3, in the presence and absence of insulin, on glucose uptake in insulin resistant L6 differentiated muscle cells after a 2 hour incubation.
FIG. 6- Chart illustrating the effect of the combination of zinc chloride and 1,25-dihydroxyvitamin D3, in the presence and absence of insulin, on glucose uptake in insulin resistant L6 differentiated muscle cells after a 2 hour incubation.
FIG. 7 - Chart illustrating the effect of zinc chloride, in the presence and absence of insulin, on glucose uptake in L6 differentiated muscle cells after a 24 hour incubation.
FIG. 8 - Chart illustrating the effect of 1,25-dihydroxyvitamin D3, in the presence and absence of insulin, on glucose uptake in L6 differentiated muscle cells after a 24 hour incubation.
FIG. 9 - Chart illustrating the effect of the combination of zinc chloride and 1,25-dihydroxyvitamin D3, in the presence and absence of insulin, on glucose uptake in L6 differentiated muscle cells after a 24 hour incubation.
FIG. 10 - Chart illustrating the effect of zinc chloride, in the presence and absence of insulin, on glucose uptake in insulin resistant L6 differentiated muscle cells after a 24 hour incubation.
FIG. 11- Chart illustrating the effect of 1,25-dihydroxyvitamin D3, in the presence and absence of insulin, on glucose uptake in insulin resistant L6 differentiated muscle cells after a 24 hour incubation.
FIG. 12- Chart illustrating the effect of the combination of zinc chloride and 1,25-dihydroxyvitamin D3, in the presence and absence of insulin, on glucose uptake in insulin resistant L6 differentiated muscle cells after a 24 hour incubation.

### Detailed Description

In a first aspect of the present invention there is provided a combination of zinc and vitamin D for use to improve insulin sensitivity and/or to treat or prevent an IGT and/or type II diabetes and/or GDM and/or to prevent a condition associated with any of the foregoing in a subject.

The term "subject" as used herein refers to a mammal and more particularly a cat, a dog or a human. The human may be an adult, child or baby including a preterm baby. In the case of GDM the term mammal refers to a pregnant mammal and/or the offspring of said pregnant mammal e.g. a pregnant cat, dog or human, or the offspring of said pregnant cat, dog or human.

Whether or not a mammal has an IGT or has type II diabetes may be determined by measuring its fasting glucose and/or HbAlc concentration, or by carrying out an oral glucose tolerance test (OGTT). The skilled person will be familiar with these tests and the criteria for diagnosing an IGT or type II diabetes. For humans the criteria for diagnosing an IGT or type II diabetes has been set out by the Standards of medical care in diabetes (2014) from the American Diabetes Association. A human subject is considered as having an IGT (prediabetic condition) if their fasting plasma-glucose concentration equates to 5.6 mmol/L or more, or if their fasting HbAlC level are between 5.7-6.4 %, or blood glucose level ranges between 7.8 -11.0 mmol/L 2 hours after a 75gram glucose drink. A human subject is considered as having type II diabetes if their fasting plasma-glucose concentration equates to 7.0 mmol/L or more, or fasting HbAlc is higher than 6.5 %, or higherthan 11.1 mmol/L 2 hours after a 75gram glucose drink.

The term "GDM" as used herein refers to any degree of impaired glucose tolerance that onsets or is first recognized during pregnancy.

Whether or not a mammal has GDM may be determined by measuring its fasting glucose plasma concentration, or by carrying out an oral glucose tolerance test (OGTT). The skilled person will be familiar with these tests and the criteria for diagnosing an impaired glucose tolerance and hence GDM. According to the criteria set out in the National Academy of clinical biochemistry (NACB) and the International Association of Diabetes and Pregnancy Study Group (IADPSG) guidelines, published by the American Association for clinical chemistry (AACC), a pregnant human subject is considered as having an GDM if their fasting plasma glucose concentration equates to 5.1mmol/L or more, or if their blood glucose concentration is higher than 10 mmol/L 1hour after a 75gram glucose drink, or higher than 8.5 mmol/L 2 hours after a 75gram glucose drink.

The term "treat" as used herein also encompasses amelioration and/or alleviation of a condition i.e. The amelioration and/or alleviation of the symptoms of a condition. It may for example encompass the reduction of the severity of a condition in a subject.

The term "prevent" as used herein refers to the prevention of the occurrence, or reduction of the risk of the occurrence, of a condition in a subject.

The term "vitamin D" as used herein refers to vitamin D, a precursor thereof, and /or a metabolite thereof.

The term "precursor" as used herein refers to any substance administered e.g. ingested by a subject and used by the body of said subject to produced and/or form a particular compound.

The term "metabolite" as used herein refers to any substance produced and/or formed by the body of a subject from a particular compound after its administration e.g. ingestion. It includes active forms and catabolites of a compound.

Any source or form of vitamin D suitable for ingestion by the subject may be used in the invention.

In particular, the vitamin D may be vitamin D2, vitamin D3, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, 1,25-dihydroxyvitamin D2, and a combination of any of the foregoing.

More particularly the vitamin D is vitamin D3, a metabolite of vitamin D selected from the group consisting of 25-hydroxyvitamin D3, 1,25-dihydroxyvitamin, and a combination of any of the foregoing. Even more particularly the vitamin D3 is Cholecalciferol.

Any source or form of zinc suitable for ingestion by the pregnant subject may be used. In particular the zinc may be a zinc salt such as zinc chloride, zinc picolinate, zinc sulfate, zinc oxide, zinc acetate, zinc carbonate, and combinations of the foregoing.

More particularly the zinc is zinc chloride.

In an embodiment a combination of zinc and vitamin D is used in combination with myo-inositol and/or one or more of vitamin B2, B6, and B12 and/or one or more probiotic.

Zinc and vitamin D with myo-inositol (*cis*-1,2,3,5-*trans*-4,6-cyclohexanehexol) and/or one or more probiotic and/or one or more of vitamin B2, B6, and B12 can be particularly effective for improving insulin sensitivity and treating and/or preventing an IGT and/or type II diabetes and/or GDM and/or preventing a condition associated with any of the foregoing in a subject. A combination of vitamin D and zinc with one or more of these listed ingredients may provide an improved effect over the combination of zinc and vitamin D alone.

Any source or form of myo-inositol suitable for ingestion by the subject may be used in the invention.

The term myo-inositol as used herein refers to myo-inositol (cis-1,2,3,5-trans-4,6-cyclohexanehexol) and /or a metabolite thereof.

A metabolite of myo-inositol can be selected from the group consisting of D-chiro-inositol and L-chiro-inositol. In particular the metabolite is D-chiro-inositol.

Any source or form of vitamins B2, B6, and B12, suitable for ingestion by the subject, may be used in the invention.

The term vitamin B12 as used herein refers to vitamin B12 and /or a metabolite thereof. The term vitamin B6 as used herein refers to vitamin B6 and /or a metabolite thereof. The term vitamin B2 as used herein refers to vitamin B2 and /or a metabolite thereof.

In particular, the vitamin B6 may be pyroxidine hydrochloride, and/or a metabolite of vitamin B6 selected from the group consisting of Pyridoxal 5'-phosphate (hereinafter PLP).

In particular, the vitamin B12 may be cyanocobalamin, and/or a metabolite of vitamin B12 selected from the group consisting of hydroxocobalamin, methylcobalamin, adenosylcobalamin, and a combination of any of the foregoing.

In particular the vitamin B2 may be riboflavin e.g. riboflavin sold under the trademark Riboflavin Universal and/or a metabolite of vitamin B2 selected from the group consisting of flavin mononucleotide (hereinafter FMN), Flavin Adenine Dinucleotide (hereinafter FAD), and salts thereof e.g. riboflavin-5'-phosphate sodium salt, and a combination of any of the foregoing.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

The one or more probiotic bacteria may be any lactic acid bacteria, Bifidobacteria, or combination thereof, wherein said lactic acid bacteria and Bifidobacteria have established probiotic characteristics. Non limiting examples of lactic acid bacteria strains include; Lactobacillus rhamnosus ATCC 53103 obtainable inter alia from Valio Oy of Finland under the trade mark LGG and Lactobacillus rhamnosus deposited by Nestle R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. None limiting examples of Bifidobacteria strains include; Bifidobacterium lactis deposited at the Collection Nationale de Cultures De Microorganismes (CNCM)as CNCM I-3446 sold inter alia by the Christian Hansen company of Denmark under the trade mark Bb12, Bifidobacterium longum ATCC BAA-999 sold by Morinaga Milk Industry Co. Ltd. of Japan under the trade mark BB536, the strain of Bifidobacterium breve sold by Danisco under the trade mark Bb-03, the strain of Bifidobacterium breve sold by Morinaga under the trade mark M-16V and the strain of Bifidobacterium breve sold by Institut Rosell (Lallemand) under the trade mark R0070.

In a particular embodiment the one or more probiotic is a mixture of Lactobacillus rhamnosus CGMCC 1.3724 and Bifidobacterium lactis CNCM I-3446

The Lactobacillus rhamnosus CGMCC 1.3724 and Bifidobacterium lactis CNCM I-3446 can be used in a ratio of 1:99 to 99:1, however, more particularly they will be used in a ratio of 1:2 to 2 to 1, even more particularly 1:1.

The combination of vitamin D and zinc, and any ingredients it is optionally in combination with e.g. the myo-inositol and/or one or more probiotic, and/or one or more of vitamin B2, B6, and B12, can be employed in any effective dose that provides a benefit with respect to improving insulin sensitivity and/or treating or preventing an IGT and/or type II diabetes and/or GDM and/or preventing a condition associated with any of the foregoing in a subject.

An effective dose may be any dose that improves, by any degree, insulin sensitivity and/or an IGT and/or type II diabetes and/or GDM in a subject.

It is well within the purview of the skilled person to determine an effective dose. An effective dose may, for example, be determined by testing the effect of a dose on a subject's fasting glucose plasma concentration, or fasting HbAlc concentration, or by carrying out an OGTT. An effective dose should improve a subject's fasting glucose plasma concentration and/or HbAlc concentration, and/or lower a subject's glycemic response.

Typically, an effective dose will depend on the type, age, size and health status of the subject, on the subject's lifestyle, as well as on its genetic heritage.

A particularly useful dose of vitamin D is 1.5 to 100 µg, or 10µg.

A particularly useful dose of zinc is 1.1 to 40 mg, or 10mg

A particularly useful dose of myo-inositol, if present, is from 0.2 to 5mg, 1.5 to 5mg, 2 to 4g.

A particularly useful dose of the one or more probiotic, if present, is from 10e5 to 10e12 colony forming units (cfu), or 10e7 to 10e11 cfu.

A particularly useful dose of vitamin B6, if present, is 0.19 to 19 mg, or 2.6mg. A particularly useful dose of vitamin B12, if present, is 0.26 to 26µg, or 5.2µg.

A particularly useful dose of vitamin B2, if present, may be 0.14 to 14 mg, 1 to 2.5mg, 1.5 to 2mg, or 1.8mg.

The term "dose" as used herein refers to a daily quantity that is administered to a subject. The daily quantity or dose may be administered all at once or it may be spread out over several administrations throughout a day. The dose may be by administered by any known method, in particularly enterally e.g. orally.

The dose(s) may be administered at any time e.g. day time or night time. The dose may be particularly effective if administered before the subject has food or beverage e.g. has a meal or a snack.

In an embodiment the dose is spread over 2 administrations, in a particular the dose is spread over 2 administrations, one in the morning and one in the evening, in particularly before breakfast and before the evening meal.

The term "breakfast" as used herein refers to the first meal of the day.

The term "evening meal" as used herein refers the last meal of the day.

The dosage requirements may be 2 administrations per day, in particular 2 administrations per day before any food or beverage. More particularly, 2 administrations per day wherein one is in the morning and one is in the evening. Even more particularly, 2 administrations per day wherein one is in the morning before breakfast and one is in the evening before the evening meal.

For the treatment and/or prevention of GDM and the prevention of a conditional associated therewith in a pregnant subject and/or its offspring, the combination of zinc and vitamin D, optionally in combination with the myo-inositol and/or one or more probiotic, and/or one or more of vitamin B2, B6, and B12, is intended for administration to a subject desiring to get pregnant, to a pregnant subject and/or to a lactating subject. In particular it is to be administered to a subject desiring to get pregnant and/or to a pregnant subject. More particularly it is for administration to a pregnant subject.

If a combination of zinc and vitamin D, optionally in combination with the myo-inositol and/or one or more probiotic, and/or one or more of vitamin B2, B6, and B12, is administered to a subject desiring to get pregnant it may for example be administered during at least 1, 2, 3 or 4 months preceding the pregnancy or desired pregnancy. If a combination of zinc and vitamin D, optionally in combination with the myo-inositol and/or one or more probiotic, and/or one or more of vitamin B2, B6, and B12 is administered to a pregnant subject, it may be administered throughout or partially throughout the pregnancy e.g. for at least 4, at least 8, at least 12, at least 16, at least 20, at least 24, at least 28, or at least 36 weeks depending on the gestational period of the subject. Administration may also continue throughout or partially throughout the lactation period of said subject.

Since the risk of GDM increases in the second and third trimester of pregnancy, administration may be particularly beneficial in the second and third trimester of pregnancy.

The vitamin D and zinc, and myo-inositol, one or more probiotic, and one or more of vitamin B2, B6, and B12 may all be provided in a format providing sustained release of said vitamins or one or more probiotic. This way, these compounds can be consumed less frequently, while the body is still constantly supplied with a sufficient amount of them.

The vitamin D and zinc may be administered separately, sequentially or simultaneously.

The vitamin D and zinc when used in combination with myo-inositol and/or one or more probiotic and/or one or more of vitamin B2, B6, and B12 may be administered in the same composition as said myo-inositol and/or one or more probiotic and/or one or more of vitamin B2, B6, and B12. Alternatively, one or more of said myo-inositol and/or one or more probiotic and/or one or more of vitamin B2, B6, and B12 may be administered separately to said vitamin D and/or zinc e.g. vitamin D and zinc (optionally with myo-inositol, and one or more of vitamin B2, B6, and B12) may be administered separately to said one or more probiotic. The separate administration may be simultaneously but separate administration e.g. vitamin D and zinc (optionally with myo-inositol, and one or more of vitamin B6, B12 and D) may be administered at the same time but separately to said one or more probiotic e.g. in two separate tablets. The vitamin D and zinc (optionally with myo-inositol, and one or more of vitamin B2, B6, and B12) may for example be administered separately and consecutively in quick succession e.g. within 5, 4, 3, 2, 1 minute, to said one or more probiotic.

Vitamin D and zinc, optionally in combination with the myo-inositol and/or one or more probiotic, and/or one or more of vitamin B2, B6, and B12, and any other optional ingredients, may be administered or employed in any form suitable for ingestion by the subject. They may for example be employed in the form of a composition e.g. a nutritional product, a food product, a functional food product, a healthy ageing product, a dairy product, a nutritional supplement, a pharmaceutical formulation, a beverage product, a diet, or a pet food product.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual.

Food products and functional food products include for example; cereal-based products, yogurts or other milk-derived products and bars.

The term "healthy ageing product" as used herein, can refer to a diet or nutritional supplement that is intended as a means to extend lifespan in an individual. Such a product may additionally contain antioxidants or other compounds such as oxytocin, insulin, human chorionic gonadotropin (hCG), erythropoietin (EPO), dietary fiber, plant sterols, PUFA, and combinations thereof.

The term "antioxidants" as used herein refers to molecules capable of slowing or preventing the oxidation of other molecules. Preferably, antioxidants are selected from: beta-carotene, vitamin C, vitamin E, selenium, carotenoids, coenzyme Q10, flavonoids, glutathione, lutein, lycopene, polyphenols, vitamin A, vitamin B1, vitamin B6, vitamin B12, vitamin C, vitamin D, vitamin E, zeaxanthin, lipoic acid, carnosine, N-acetylcysteine, or combinations thereof.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids. Nutritional supplements can for example be provided in the form of a pill, a tablet, a lozenger, a chewy capsule or tablet, a tablet or capsule, or a powder supplement that can for example be dissolved in an aqueous medium e.g. water or juice, or sprinkled on food. Such supplements typically provide the selected nutrients while not representing a significant portion of the overall nutritional needs of the subject. Typically they do not represent more than 0.1%, 1%, 5%, 10% or 20% of the daily energy need of the subject. A specific type of nutritional supplement is a pre pregnancy and/or pregnancy and/or lactation (breast feeding) supplement.

Powdered nutritional supplements that can for example be sprinkled on food or dissolved in an aqueous medium e.g. water, juice or milk, are currently well accepted by consumers.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art.

The term "beverage product" as used herein, refers to a nutritional product in liquid or semiliquid form that may be safely consumed by an individual.

The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

Vitamin D and zinc, optionally in combination with myo-inositol and/or one or more probiotic, and/or one or more of vitamin B2, B6, and B12, may also be used in combination with other vitamins and minerals. e.g. calcium, magnesium, phosphorus, iron, copper, iodine, selenium, vitamin A or retinol activity equivalent (RAE) e.g. beta carotene or a mix of carotenoids, Vitamin C, Vitamin B1, niacin, folic acid, biotin, Vitamin E.

It may be particularly beneficial if vitamin D and zinc, optionally in combination with myo-inositol and/or one or more probiotic, and or one or more of vitamin B2, B6, and B12, is used in combination with one or more of the following micronutrients in the following amounts: 100 to 2500 mg calcium, 35 to 350 mg magnesium, 70 to 3500 mg phosphorus, 2.7 to 45 mg iron, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg Vitamin C, 0.14 to 14 mg Vitamin B1, 1.8 to 35 mg niacin, 60 to 1000 µg folic acid, 3 to 300 µg biotin, 1.9 to 109 µg Vitamin E.

In a particular embodiment vitamin D and zinc is used in combination with myo-inositol, one or more probiotic, vitamin B2, B6, and B12, B-carotene, folic acid, iron, calcium, and iodine.

Vitamin D and zinc, optionally in combination with myo-inositol and/or one or more probiotic, and/or one or more of vitamin B2, B6, and B12, may also be advantageously used in combination with at least one oligosaccharide and/or at least one long chain polyunsaturated fatty acids, such as arachidonic acid (ARA), eicosapentaenoic acid (EPA) and/or docosahexaenoic acid (DHA). The oligosaccharides and long chain polyunsaturated fatty acids can be used in any concentration safe for administration to the subject.

Vitamin D and zinc, optionally in combination with the myo-inositol and/or one or more probiotic, and/or one or more of vitamin B2, B6, and B12, may also be used in combination with other ingredients commonly used in the form of a composition in which it is employed e.g powdered nutritional supplement, a food product, or a dairy product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavour agents e.g. cocoa powder or honey, osmotic agents, pharmaceutically acceptable carriers, preservatives, sugars, sweeteners, texturizers, emulsifiers, water and any combination thereof.

However, notwithstanding the above, a high fat intake can be undesirable and is linked to an increased risk of excess weight and obesity. Accordingly, in a particular embodiment the vitamin D and zinc, optionally in combination with myo-inositol and/or one or more probiotic, and/or one or more of vitamin B2, B6, and B12 is not used in combination with a long chain polyunsaturated fatty acid and/or animal fats and/or vegetable fats.

In another particular embodiment the vitamin D and zinc, optionally in combination with myo-inositol and/or one or more probiotic, and/or one or more of vitamin B2, B6, and B12 is not used in combination with an oligosaccharide.

In a particular embodiment a combination of zinc and vitamin D is used only in combination with myo-inositol.

In particular embodiment a combination of zinc and vitamin D is used only in combination with one or more of vitamin B2, B6, and B12.

In particular embodiment a combination of zinc and vitamin D is used only in combination with one or more probiotic.

In a particular embodiment a combination of zinc and vitamin D is used only in combination with myo-inositol and one or more of vitamin B2, B6, and B12.

In a particular embodiment a combination of zinc and vitamin D is used only in combination with myo-inositol and with one or more probiotic.

In a particular embodiment a combination of zinc and vitamin D is used only in combination with one or more of vitamin B2, B6, and B12 and with one or more probiotic.

In a particular embodiment a combination of zinc and vitamin D is used only in combination with myo-inositol, one or more probiotic, vitamin B2, B6, and B12, B-carotene, folic acid, iron, calcium, and iodine.

In an embodiment vitamin D and zinc are not used in combination with any other compound.

As stated hereinabove, a low insulin sensitivity and/or an IGT and/or type II diabetes in a subject is associated with a variety of conditions that affect the subject e.g. CVD. A low insulin sensitivity and/or type II diabetes is considered as being associated with a condition if it increases the risk of a subject having or developing that condition during its lifetime.

Non limiting examples of conditions associated with a low insulin sensitivity and/or an IGT, and/or type II diabetes include: CVD, strokes, circulatory problems that in extreme cases can lead to amputation, diabetic retinopathy, kidney failure, hearing loss, and impaired cognitive ability.

GDM is considered as being associated with a condition if it increases the risk of a subject having or developing that condition during pregnancy, during birth, after birth, or later in the life of said subject, said subject being the pregnant subject or its offspring.

Non limiting examples of conditions associated with GDM, that affect the pregnant subject and/or its offspring, include; preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity, immediately after birth and later in the life of the offspring, and an increased risk for the mother of having or developing type 2 diabetes immediately after birth and later in life.

A low insulin sensitivity, an IGT and type II diabetes are more prevalent amongst subjects who are overweight or obese, or if there is a family history of type II diabetes.

Accordingly, a combination of vitamin D and zinc, optionally in combination with the myo-inositol and/or one or more probiotic, and/or one or more of vitamin B2, B6, and B12, may be particularly beneficial for improving a low insulin sensitivity and/or treating or preventing an IGT, type II diabetes and/ or preventing conditions associated with any of the foregoing in one or more of these subsets of subjects.

GDM is more prevalent amongst subjects who suffered with it in a previous pregnancy, if the subject is overweight or obese, if there is a family history of GDM or type II diabetes. If the subject is human and over 25 years of age, and if the subject is human and is black, Hispanic, American Indian or Asian.

Accordingly, a combination of zinc and vitamin D, optionally in combination with the myo-inositol and/or one or more probiotic, and or one or more of vitamin B2, B6, and B12, may be particularly beneficial for treating and/or preventing GDM in one or more of these subsets of subjects.

In a particular embodiment the combination of zinc and vitamin D is used in combination with vitamin D and zinc, myo-inositol, one or more probiotic, vitamin B2, B6, and B12, B-carotene, folic acid, iron, calcium, and iodine and the one or more probiotic is preferably a mixture of Lactobacillus rhamnosus CGMCC 1.3724 and Bifidobacterium lactis CNCM 1-3446.

Useful concentrations in which these ingredients may be used or employed in a composition are set out hereinabove.

Further ingredients that may be used in combination with zinc and vitamin D, and therefore contained in said composition are also set out above.

The composition may be a nutritional product, a food product, a functional food product, a healthy ageing product, a dairy product, a nutritional supplement, a pharmaceutical formulation, a beverage product, a diet, or a pet food product. In a particular embodiment the composition is a maternal supplement for use in pre-pregnancy and/or pregnancy and/or during lactation.

As would be clear to the skilled person, the composition may be for use to improve insulin sensitivity and/or to treat or prevent an impaired glucose tolerance and/or type II diabetes and/or gestational diabetes mellitus and/or to prevent a condition associated with any of the foregoing in a subject.

As would be clear to the skilled person, all features and embodiments disclosed in combination with the combination of zinc and vitamin D for use to improve insulin sensitivity and/or treat or prevent an IGT, and/or type II diabetes and/or GDM and/or to prevent a condition associated with any of the foregoing in a subject, are equally valid in respect to said composition e.g. said composition may comprise any ingredient with which zinc and vitamin D may be used, as set out herein. As the skilled person will appreciate, a composition comprising zinc and vitamin D may replace the combination of zinc and vitamin D in any method or use set out herein

Those skilled in the art will understand that they can freely combine all features of the present invention disclosed herein. In particular, features described for different embodiments of the present invention may be combined. Where known equivalents exist to specific features, such equivalents are incorporated as if specifically referred to in this specification. Further advantages and features of the present invention are apparent from the figure and non-limiting example.

The present invention will now be described in further details by the way of the following examples.

### Examples

### Example 1

An example composition comprising a combination of zinc and vitamin D in combination with myo-inositol, vitamin B2, B6, and B12, and Lactobacillus rhamnosus GG¹ and Bifidobacterium lactis BB12² is set out in table 1.

The composition in table I is for a nutritional supplement in a powder form, intended to be sprinkled on food.

**Table 1 : Composition of Example 1**

| **Ingredient** | **Amount per daily dose** |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG¹⁾ | 1x10⁹ cfu |
| Bifidobacterium lactis BB12²⁾ | 1x10⁹ cfu |

1) Strain deposited as CGMCC 1.3724
2) Strain deposited as CNCM I-3446

The composition may be provided as a kit of parts comprising in one sachet the probiotic as a powder and in a second sachet all other ingredients.

### Example 2

A milk powder drink for pregnant women to be reconstituted in water is provided consisting of 30g of milk powder per serving admixed with the ingredients listed in Table 1, in half of the amounts mentioned in Table 1. The composition is to be administered to a pregnant woman twice per day during at least the third trimester of pregnancy.

### Example 3

The effect of zinc and vitamin D on glucose uptake in L6 differentiated muscle cells was measured after short (2hours) and long term (24 hours) incubations.

Ingredient J refers to zinc chloride and ingredient K refers to 1,25-dihydroxyvitamin D3.

Ingredient J was tested at 10 µM and 20 µM.

Ingredient K was tested at 1 nM and 10 nM.

Ingredient J was prepared in water and stored at -20°C (mother solution at 2 M) and ingredient K was prepared in DMSO and stored at -20°C (mother solution at 0.01 mM). The day of the experiment, the ingredients were diluted in a serum-free medium (DMEM) containing 5.5 mM D-glucose to achieve the final tested concentrations and 0.1 % of DMSO.

L6 myoblasts were maintained in growth medium or DMEM 25 mM glucose supplemented with 10 % of fetal bovine serum (FBS), 100 U/ml penicillin, and 100 µg/ml streptomycin. For the glucose uptake study, cells were grown on 24 well plates at a density of 1000-1500 cells/well in 0.5 mL of growth medium. Five days after plating, the differentiation induction into myotubes was carried out in DMEM 25 mM glucose containing 2 % FBS, 100 U/ml penicillin, and 100 µg/ml streptomycin at 37°C under a 5 % CO₂ atmosphere. The culture medium was changed every other day.

For short term incubation (2 hours), the day prior to the test, the culture medium was replaced by DMEM 25 mM D-glucose containing 0.25 % BSA, 100 U/ml penicillin, and 100 µg/ml streptomycin. The day after and prior to the test, cells were washed and incubated in DMEM containing 5.5 mM D-glucose, 0.34 % BSA in the presence of the ingredients for 2 hours with and without insulin (100 nM). To mimic physiopathological conditions and to create insulin resistance, some cells were exposed to the free fatty acid Palmitate (PALM) for 6 hours before glucose uptake measurement.

For long term incubation (24 hours), the day prior to the test, cells were pre-incubated in the presence of the tested ingredients (ingredient J, K, or a combination thereof) for 18 hours in DMEM 25 mM D-glucose containing 0.25 % BSA, 100 U/ml penicillin, and 100 µg/ml streptomycin in the absence of insulin. The day after and prior to the test, cells were washed and incubated in the presence of the tested ingredients for 6 hours more in DMEM containing 5.5 mM D-glucose, 0.34 % BSA without insulin. Insulin (100 nM) was added for the last 2 hours.

In normal and physiopathological conditions, glucose uptake was measured by incubation of the cells with radiolabelled 2-deoxy-D-[1,2 3H] glucose for 15 min. Glucose uptake was arrested by two washing steps in ice-cold PBS 1X. Then cells were solubilized in 0.1 N NaOH for 30 min. Cell associated radioactivity was then counted and protein quantification was determined using the coloric Lowry method.

Each condition was tested in triplicate. Insulin (100 and 1000 nM / 2 hours) and Metformin (1.5 mM / 24 hours) were used as positive controls.

Results are expressed in pmol glucose incorporated / min / mg protein and in % of control or basal condition or insulin 0 nM (100 %).

A statistical analysis allowing multiple comparisons (one way ANOVA followed by a Dunnett's t test; * *P* < 0.05, ***P* < 0.01, ****P* < 0.001 versus control condition) was carried out. All ingredients showing significant increase in glucose uptake compared to control or basal condition are considered active.

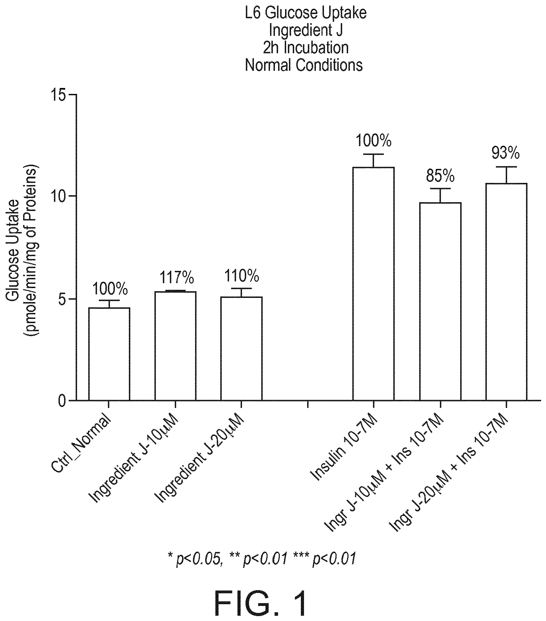

The results are shown in Figures 1 to 12.

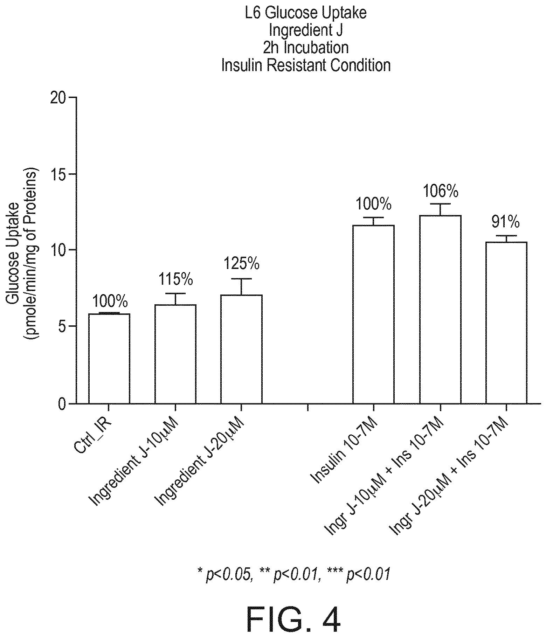

As can be seen from figure 1 and/or figure 4, no increase or a slight but not significant increase in glucose uptake in L6 differentiated muscle cells, or insulin resistant L6 differentiated muscle cells, was observed with ingredient J after short term incubation (2 hrs) in the presence or absence of insulin.

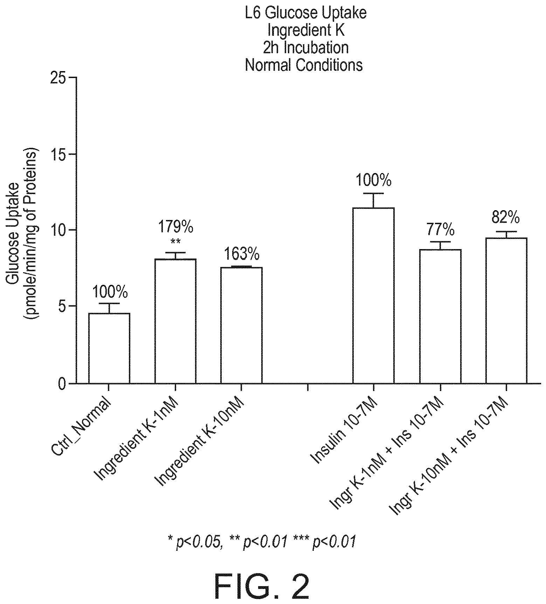

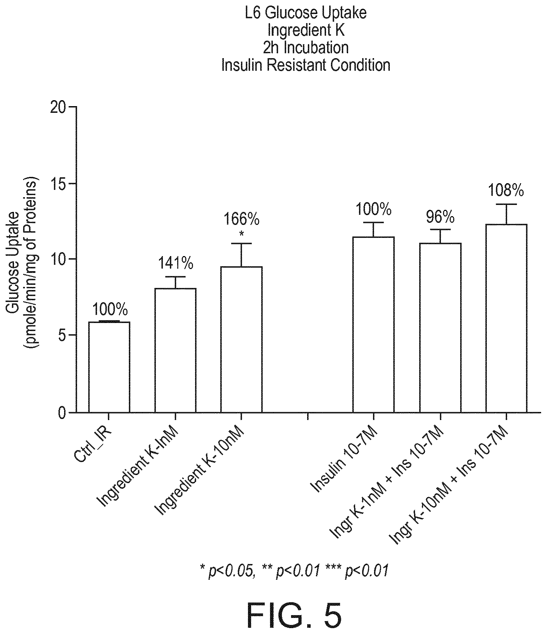

As can be seen from figure 2 and/or figure 5, significant increases in glucose uptake were observed in L6 differentiated muscle cells, and insulin resistant L6 differentiated muscle cells, with ingredient K after short term incubation (2 hrs) in the absence of insulin. In the presence of insulin (100 nM), ingredient K did not increase insulin-induced glucose uptake.

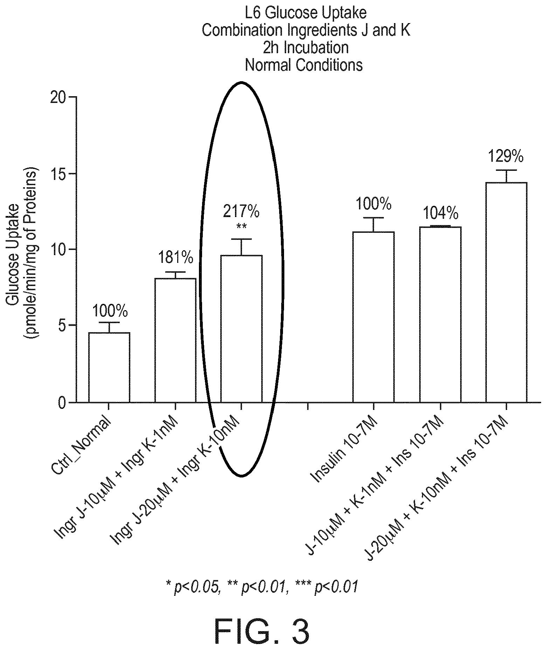

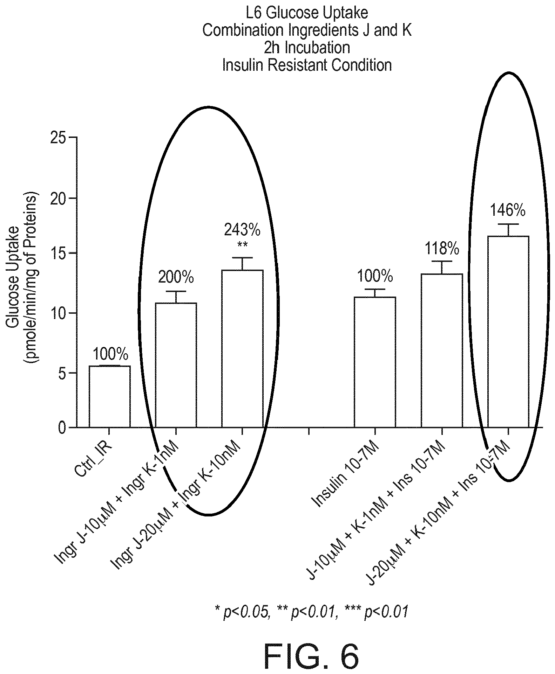

As can be seen from figure 3 and/or figure 6, significant increases in glucose uptake were observed in L6 differentiated muscle cells, and insulin resistant L6 differentiated muscle cells, with the combination of ingredient K and ingredient J after short term incubation (2 hrs) in the absence of insulin. The increases in glucose uptake observed with the combination of ingredient K and ingredient J were greater than the additive values of each ingredient tested separately.

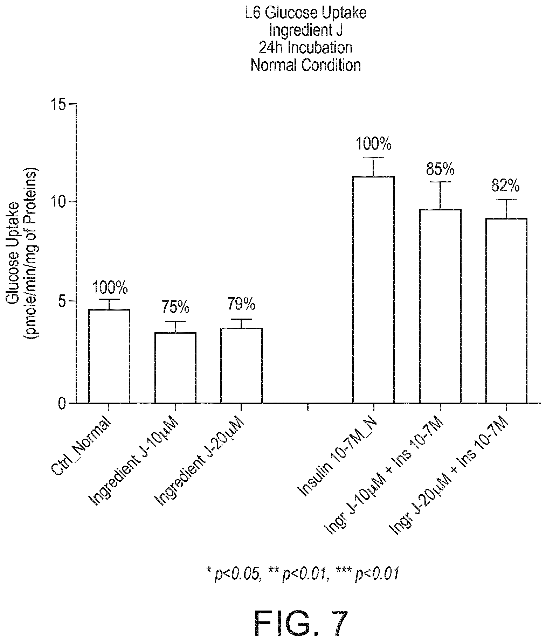

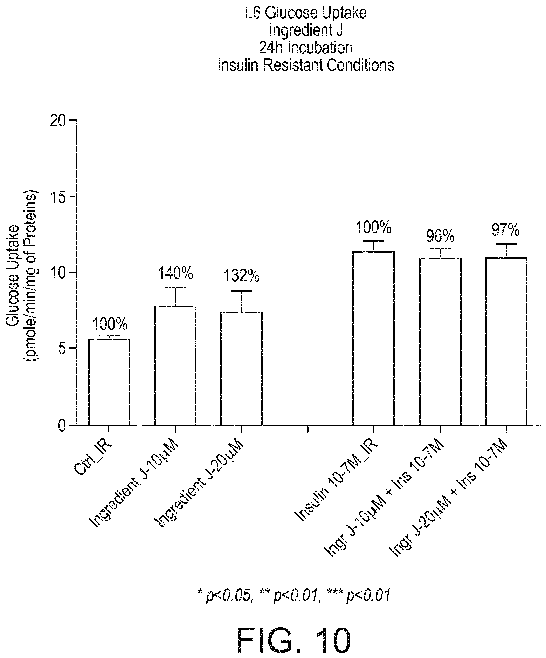

As can be seen from figure 7 no increase or a slight but not significant increase in glucose uptake in L6 differentiated muscle cells was observed with ingredient J after long term incubation (24 hrs) in the presence or absence of insulin. As can be seen from figure 10 no increase or a slight but not significant increase in glucose uptake in insulin resistant L6 differentiated muscle cells was observed with ingredient J after long term incubation (24 hrs) in the presence or absence of insulin.

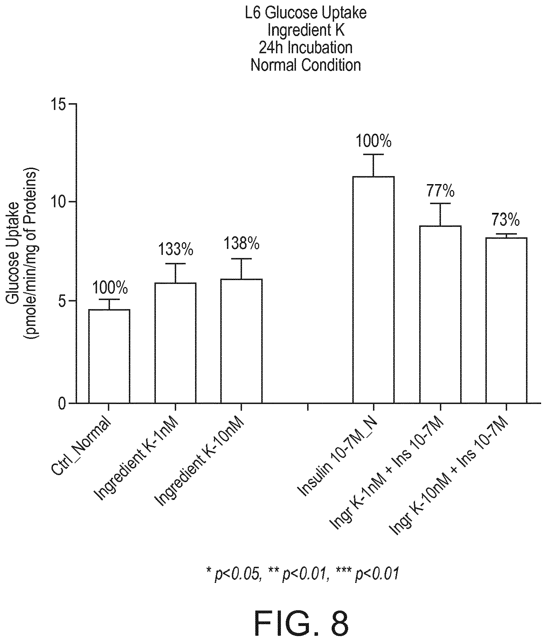

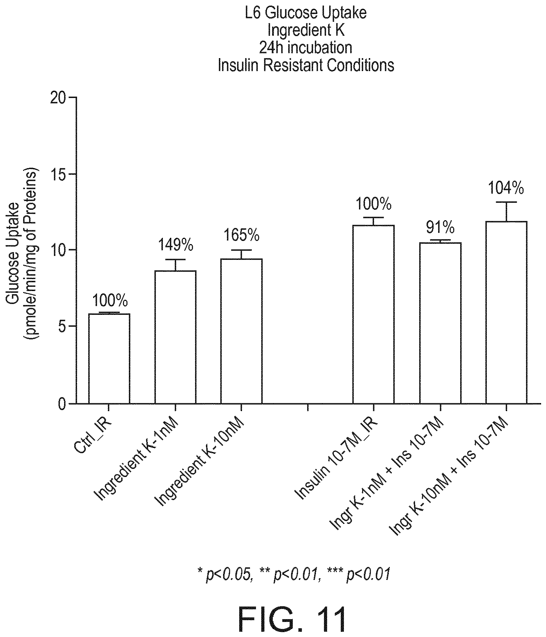

As can be seen from figure 8 and 11 no increases or a slight but not significant increase in glucose uptake in L6 differentiated muscle cells, and insulin resistant L6 differentiated muscle cells, was observed with ingredient K after long term incubation (24 hrs) in the absence of insulin. No increase or a slight but not significant increase in glucose uptake in L6 differentiated muscle cells, and insulin resistant L6 differentiated muscle cells, was observed with ingredient K after long term incubation (24 hrs) in the presence of insulin.

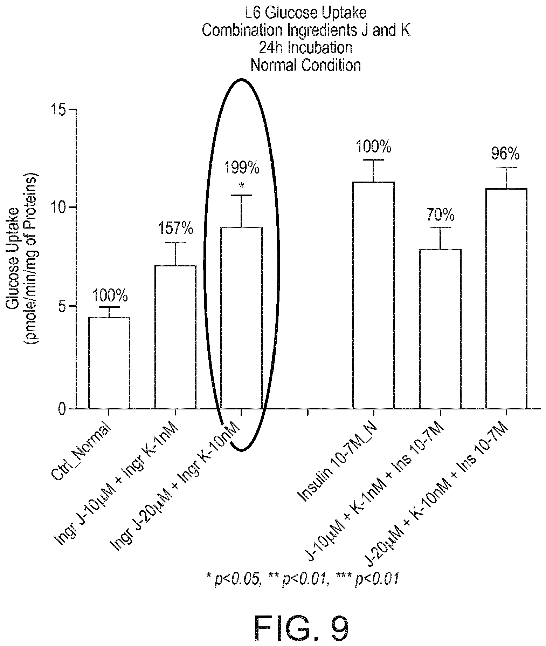

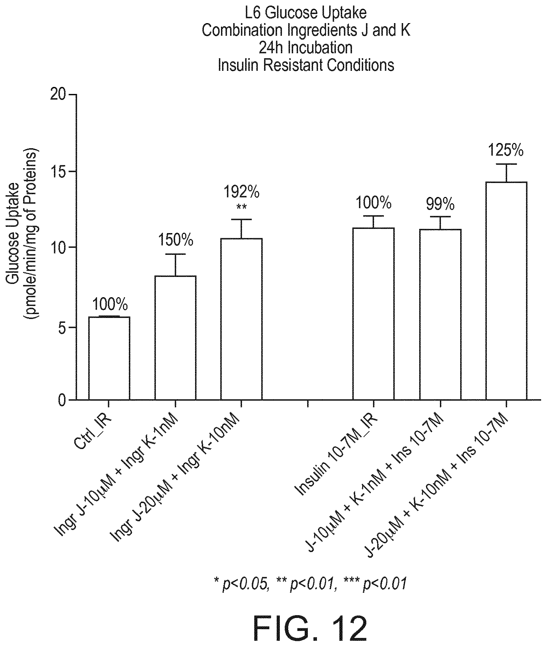

As can be seen from figure 9 and/or figure 12, significant increases in glucose uptake were observed in L6 differentiated muscle cells, and insulin resistant L6 differentiated muscle cells, with the combination of ingredient K and ingredient J after long term incubation (24 hrs) in the absence of insulin. In the presence of insulin a slight but not significant increase in glucose uptake was observed in insulin resistant L6 differentiated muscle cells, with the combination of ingredient K and ingredient J after long term incubation (24 hrs).

The increases in glucose uptake observed with the combination of ingredient K and ingredient J were greater than the additive values of each ingredient tested separately.

## Claims

1. A combination of zinc and vitamin D for use to improve insulin sensitivity and/or to treat or prevent an impaired glucose tolerance and/or type II diabetes and/or gestational diabetes mellitus (GDM) and/or to prevent a condition associated with the foregoing in a subject
wherein said zinc and vitamin D are administered simultaneously or separately and optionally sequentially wherein, the condition associated with GDM is selected from preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity immediately after birth and later in the life of the offspring, and an increased risk for the mother of having or developing type 2 diabetes immediately after birth and later in life.

2. A combination of zinc and vitamin D according to claim 1 for use as defined in claim 1, wherein the
combination of zinc and vitamin D is used in combination with myo-inositol and/or one or more of vitamin B2, B6, and B12 and/or one or more probiotic preferably selected from the group consisting of; Lactobacillus, bifidobacterium, and combinations thereof, and more preferably selected from the group consisting of lactobacillus rhamnosus GG (CGMCC 1.3724), bifidobacterium lactus BB-12 (CNCM 1-3446), and a combination thereof.

3. A combination of zinc and vitamin according to claim 1 or 2 for use as defined in claim 1 or 2, wherein the zinc is used in a concentration of 1.1 to 40 mg per daily dose and the vitamin D is used in a concentration of 1.5 to 100 µg, myo-inositol if used is used in a concentration of 0.2 to 5 mg per daily dose, vitamin B6 if used is used in a concentration of 0.14 to 14 mg per daily dose, B12 if used is used in an amount of 0.26 to 26 µg per daily dose, B2 if used is used in an amount of 0.14 to 14 mg per daily dose, and wherein
the one or more probiotic if used is used in a concentration of 10e5 to 10e12 colony forming units (cfu) per daily dose.

4. A combination of zinc and vitamin D according to anyone of claims 1 to 3 for use as defined in any one of claims 1 to 3, wherein the subject is a mammal selected from the group consisting of; a cat, a dog, a human, and wherein said combination of zinc and vitamin D is preferably administered
enterally, more preferably orally, and preferably in the form of a nutritional product, a food product, a functional food product, a healthy ageing product, a dairy product, a nutritional supplement, a pharmaceutical formulation, a beverage product, a diet, or a pet food product.

5. A combination of zinc and vitamin D according to anyone of claims 1 to 3 for use as defined in any one of claims 1 to 3, wherein the use is for the treatment or prevention of gestational diabetes mellitus and/or the prevention of a condition associated therewith, the subject is a pregnant mammal or the offspring of a pregnant mammal, and wherein said combination of zinc and vitamin D is preferably administered enterally, more preferably orally, and preferably in the form of a nutritional product, a food product, a functional food product, a healthy ageing product, a dairy product, a nutritional supplement, a pharmaceutical formulation, a beverage product, a diet, or a pet food product.

6. A combination of zinc and vitamin D according to anyone of claims 1 to 5 for use as defined in anyone of claims 1 to 5 wherein the combination of zinc and vitamin D is used in combination with one or more probiotic and wherein the
combination also comprises myo-inositol, and optionally one or more of vitamin B2,vitamin B6, vitamin B12, vitamin D, B-carotene, folic acid, Iron, calcium, iodine, and wherein the one or more probiotic is a mixture of Lactobacillus rhamnosus CGMCC 1.3724 and Bifidobacterium lactis CNCM 1-3446.

## Patentansprüche

1. Kombination aus Zink und Vitamin D zur Verwendung, um die Insulinsensitivität zu verbessern und/oder eine eingeschränkte Glucosetoleranz und/oder Typ II-Diabetes und/oder Schwangerschaftsdiabetes mellitus (GDM) zu behandeln oder zu verhindern und/oder um ein Leiden im Zusammenhang mit den Vorhergehenden in einem Subjekt zu verhindern, wobei das Zink und Vitamin D gleichzeitig oder getrennt und wahlweise nacheinander verabreicht werden, wobei das Leiden in Zusammenhang mit GDM ausgewählt ist aus Frühgeburt und Kaiserschnitt, Geburtsverletzung der Mutter oder des Kindes, Schulterdystokie, Makrosomie, übermäßiger Blutzuckerkonzentration der Nachkommen, übermäßigem Gewicht/Adipositas und im Zusammenhang stehenden metabolischen Störungen, z. B. Typ II-Diabetes, Fettleber und Fettleibigkeit unmittelbar nach der Geburt und später im Leben der Nachkommen, und einem erhöhten Risiko für die Mutter, Typ 2-Diabetes unmittelbar nach der Geburt und später im Leben zu haben oder zu entwickeln.

2. Kombination aus Zink und Vitamin D nach Anspruch 1 zur Verwendung wie in Anspruch 1 definiert, wobei die Kombination aus Zink und Vitamin D in Kombination mit Myoinosit und/oder einem oder mehreren von Vitamin B2, B6 und B12 und/oder einem oder mehreren Probiotika, vorzugsweise ausgewählt aus der Gruppe, bestehend aus; Lactobacillus, Bifidobacterium und Kombinationen davon, und mehr bevorzugt ausgewählt aus der Gruppe, bestehend aus Lactobacillus rhamnosus GG (CGMCC 1.3724), Bifidobacterium lactus BB-12 (CNCM I-3446) und einer Kombination davon, verwendet wird.

3. Kombination aus Zink und Vitamin nach Anspruch 1 oder 2 zur Verwendung wie in Anspruch 1 oder 2 definiert, wobei das Zink in einer Konzentration von 1,1 bis 40 mg pro Tagesdosis verwendet wird und das Vitamin D in einer Konzentration von 1,5 bis 100 µg verwendet wird, Myoinosit, wenn verwendet, in einer Konzentration von 0,2 bis 5 mg pro Tagesdosis verwendet wird, Vitamin B6, wenn verwendet, in einer Konzentration von 0,14 bis 14 mg pro Tagesdosis verwendet wird, B12, wenn verwendet, in einer Menge von 0,26 bis 26 µg pro Tagesdosis verwendet wird, B2, wenn verwendet, in einer Menge von 0,14 bis 14 mg pro Tagesdosis verwendet wird, und wobei das eine oder die mehreren Probiotika, wenn verwendet, in einer Konzentration von 10e5 bis 10e12 koloniebildenden Einheiten (KBE) pro Tagesdosis verwendet werden.

4. Kombination aus Zink und Vitamin D nach einem der Ansprüche 1 bis 3 zur Verwendung wie in einem der Ansprüche 1 bis 3 definiert, wobei das Subjekt ein Säuger ist, ausgewählt aus der Gruppe, bestehend aus; einer Katze, einem Hund, einem Menschen, und wobei die Kombination aus Zink und Vitamin D vorzugsweise enteral, mehr bevorzugt oral, und vorzugsweise in Form eines Ernährungsproduktes, eines Lebensmittelproduktes, eines funktionellen Lebensmittelproduktes, eines Produktes für gesundes Altern, eines Milchproduktes, eines Nahrungsergänzungsmittels, einer pharmazeutischen Formulierung, eines Getränkeproduktes, einer Diät oder eines Haustierfutterproduktes verabreicht wird.

5. Kombination aus Zink und Vitamin D nach einem der Ansprüche 1 bis 3 zur Verwendung wie in einem der Ansprüche 1 bis 3 definiert, wobei die Verwendung zur Behandlung oder Verhinderung von Schwangerschaftsdiabetes mellitus und/oder die Verhinderung eines damit in Zusammenhang stehenden Leidens ist, das Subjekt ein schwangerer Säuger oder der Nachkomme eines schwangeren Säugers ist, und wobei die Kombination aus Zink und Vitamin D vorzugsweise enteral, mehr bevorzugt oral, und vorzugsweise in Form eines Ernährungsproduktes, eines Lebensmittelproduktes, eines funktionellen Lebensmittelproduktes, eines Produktes für gesundes Altern, eines Milchproduktes, eines Nahrungsergänzungsmittels, einer pharmazeutischen Formulierung, eines Getränkeproduktes, einer Diät oder eines Haustierfutterproduktes verabreicht wird.

6. Kombination aus Zink und Vitamin D nach einem der Ansprüche 1 bis 5 zur Verwendung wie in einem der Ansprüche 1 bis 5 definiert, wobei die Kombination aus Zink und Vitamin D in Kombination mit einem oder mehreren Probiotika verwendet wird, und wobei die Kombination auch Myoinosit und wahlweise eines oder mehrere von Vitamin B2, Vitamin B6, Vitamin B12, Vitamin D, B-Karotin, Folsäure, Eisen, Kalzium, Iod umfasst, und wobei das eine oder die mehreren Probiotika eine Mischung aus Lactobacillus rhamnosus CGMCC 1.3724 und Bifidobacterium lactis CNCM 1-3446 ist.

## Revendications

1. Combinaison de zinc et de vitamine D pour une utilisation pour améliorer la sensibilité à l'insuline et/ou pour traiter ou prévenir une intolérance au glucose et/ou un diabète de type II et/ou un diabète sucré gestationnel (GDM) et/ou pour prévenir une affection associée à ce qui précède chez un sujet dans laquelle ledit zinc et ladite vitamine D sont administrés simultanément ou séparément et éventuellement de façon séquentielle, l'affection associée au GDM est choisie parmi un accouchement avant terme et par césarienne, une lésion obstétricale de la mère ou du bébé, une dystocie des épaules, une macrosomie, une concentration excessive en glucose dans le sang de la progéniture, un excès de poids/une adiposité et des troubles métaboliques associés par exemple au diabète de type II, une maladie du foie gras et une obésité immédiatement après la naissance et plus tard dans la vie de la progéniture, et un risque accru pour la mère d'avoir ou de développer un diabète de type 2 immédiatement après la naissance et plus tard dans la vie.

2. Combinaison de zinc et de vitamine D selon la revendication 1 pour une utilisation telle que définie dans la revendication 1, dans laquelle la combinaison de zinc et de vitamine D est utilisée en combinaison avec du myo-inositol et/ou une ou plusieurs parmi les vitamines B2, B6 et B12 et/ou un ou plusieurs probiotiques choisis de préférence dans le groupe constitué de ; Lactobacillus, bifidobacterium, et des combinaisons de ceux-ci, et plus préférablement choisis dans le groupe constitué de lactobacillus rhamnosus GG (CGMCC 1.3724), bifidobacterium lactus BB-12 (CNCM I-3446), et une combinaison de ceux-ci.

3. Combinaison de zinc et de vitamine selon la revendication 1 ou 2 pour une utilisation telle que définie dans la revendication 1 ou 2, dans laquelle le zinc est utilisé en une concentration de 1,1 à 40 mg par dose journalière et la vitamine D est utilisée en une concentration de 1,5 à 100 µg, le myo-inositol, s'il est utilisé, est utilisé en une concentration de 0,2 à 5 mg par dose journalière, la vitamine B6, si elle est utilisée, est utilisée en une concentration de 0,14 à 14 mg par dose journalière, la B12, si elle est utilisée, est utilisée en une quantité de 0,26 à 26 µg par dose journalière, la B2, si elle est utilisée, est utilisée en une quantité de 0,14 à 14 mg par dose journalière, et dans laquelle le ou les probiotiques, s'ils sont utilisés, sont utilisés en une concentration de 10e5 à 10e12 unités formant des colonies (ufc) par dose journalière.

4. Combinaison de zinc et de vitamine D selon l'une quelconque des revendications 1 à 3 pour une utilisation telle que définie dans l'une quelconque des revendications 1 à 3, dans laquelle le sujet est un mammifère choisi dans le groupe constitué de ; un chat, un chien, un humain, et dans laquelle ladite combinaison de zinc et de vitamine D est de préférence administrée par voie entérale, plus préférablement par voie orale, et de préférence sous la forme d'un produit nutritionnel, d'un produit alimentaire, d'un produit alimentaire fonctionnel, d'un produit pour un vieillissement en bonne santé, d'un produit laitier, d'un complément nutritionnel, d'une formulation pharmaceutique, d'un produit de boisson, d'un régime alimentaire, ou d'un produit alimentaire pour animal de compagnie.

5. Combinaison de zinc et de vitamine D selon l'une quelconque des revendications 1 à 3 pour une utilisation telle que définie dans l'une quelconque des revendications 1 à 3, dans laquelle l'utilisation est pour le traitement ou la prévention d'un diabète sucré gestationnel et/ou la prévention d'une affection qui lui est associée, le sujet est un mammifère gravide ou la progéniture d'un mammifère gravide, et dans laquelle ladite combinaison de zinc et de vitamine D est de préférence administrée par voie entérale, plus préférablement par voie orale, et de préférence sous la forme d'un produit nutritionnel, d'un produit alimentaire, d'un produit alimentaire fonctionnel, d'un produit pour un vieillissement en bonne santé, d'un produit laitier, d'un complément nutritionnel, d'une formulation pharmaceutique, d'un produit de boisson, d'un régime alimentaire, ou d'un produit alimentaire pour animal de compagnie.

6. Combinaison de zinc et de vitamine D selon l'une quelconque des revendications 1 à 5, pour une utilisation telle que définie dans l'une quelconque des revendications 1 à 5, dans laquelle la combinaison de zinc et de vitamine D est utilisée en combinaison avec un ou plusieurs probiotiques et dans laquelle la combinaison comprend également du myo-inositol, et éventuellement un ou plusieurs parmi la vitamine B2, la vitamine B6, la vitamine B12, la vitamine D, le B-carotène, l'acide folique, du fer, du calcium, de l'iode, et dans laquelle le ou les probiotiques sont un mélange de Lactobacillus rhamnosus CGMCC 1.3724 et de Bifidobacterium lactis CNCM 1-3446.
